# EUROPEAN PATENT APPLICATION

(11) **EP 1 995 327 A1**
(43) Date of publication of application: **26.11.2008**
(21) Application number: 07010066.4
(22) Date of filing: 21.05.2007
(51) Int. Cl.: C12Q 1/68

(54) **Probe for detecting a particular nucleic acid sequence**

(71) Applicant: Humboldt Universität zu Berlin, 10099 Berlin (DE)
(72) Inventor: Seitz, Oliver, 10405 Berlin (DE); Grossmann, Tom N., 12623 Berlin (DE); Röglin, Lars, Dr., 45144 Essen (DE)
(74) Representative: Czychowski, Christian A.

(57) **Abstract**

The invention relates to a detection molecule (triplex Molecular Beacon, tMB) for detecting a particular nucleic acid sequence, in particular for detecting a single nucleotide mismatch in a target nucleic acid. Such a detection molecule comprises a) an oligonucleotide, and b) a probe comprising a first stem forming portion with a fluorophore for generating a detection signal, a second stem forming portion with a quencher for quenching the detection signal from the fluorophore when the quencher is in proximity to the fluorophore, and a loop forming portion located between the first and the second stem forming portion, for allowing the first stem forming portion to form a stem structure together with the second stem forming portion of the probe and with the oligonucleotide. According to the invention, the oligonucleotide comprises a portion allowing it to form a triplex structure with the first and second stem forming portions of the probe.

SEQUENCE LISTING

<110>  Humboldt-Universität zu Berlin

<120>  Probe for detecting a particular nucleic acid sequence

<130>  FBP18649

<160>  24

<170>  PatentIn version 3.3

<210>  1
<211>  31
<212>  DNA
<213>  Artificial

<220>
<223>  Oligonucleotide

<400>  1
ttttttttaa gttaagacct atgttttttt t                                    31

<210>  2
<211>  37
<212>  DNA
<213>  Artificial

<220>
<223>  Oligonucleotide

<400>  2
tttttttttt taagttaaga cctatgtttt ttttttt                             37

<210>  3
<211>  31
<212>  DNA
<213>  Artificial

<220>
<223>  Oligonucleotide

<220>
<221>  misc_feature
<222>  (1)..(1)
<223>  5' 6-FAM

<220>
<221>  misc_feature
<222>  (31)..(31)
<223>  3' Dabcyl

<400>  3
ttttttttaa gttaagacct atgttttttt t                                    31

<210>  4
<211>  37
<212>  DNA
<213>  Artificial

<220>
<223>  Oligonucleotide


<220>
<221>  misc_feature
<222>  (1)..(1)
<223>  5' 6-FAM

<220>
<221>  misc_feature
<222>  (37)..(37)
<223>  3' Dabcyl

<400>  4
tttttttttt taagttaaga cctatgtttt ttttttt                                37


<210>  5
<211>  15
<212>  DNA
<213>  Artificial

<220>
<223>  Oligonucleotide

<400>  5
cataggtctt aactt                                                        15


<210>  6
<211>  15
<212>  DNA
<213>  Artificial

<220>
<223>  Oligonucleotide

<400>  6
cataggtgtt aactt                                                        15


<210>  7
<211>  9
<212>  PRT
<213>  Artificial

<220>
<223>  Oligonucleotide


<220>

<221>  MISC_FEATURE
<222>  (1)..(1)
<223>  Ac-NH-aaaaaa

<400>  7

Leu Tyr Ser Leu Tyr Ser Gly Leu Tyr
1               5


<210>  8
<211>  9
<212>  PRT
<213>  Artificial

<220>
<223>  Oligonucleotide


<220>
<221>  MISC_FEATURE
<222>  (1)..(1)
<223>  Ac-NH-aaaaaaa

<400>  8

Leu Tyr Ser Leu Tyr Ser Gly Leu Tyr
1               5


<210>  9
<211>  9
<212>  PRT
<213>  Artificial

<220>
<223>  Oligonucleotide


<220>
<221>  MISC_FEATURE
<222>  (1)..(1)
<223>  Ac-NH-aaaaaaaa

<400>  9

Leu Tyr Ser Leu Tyr Ser Gly Leu Tyr
1               5


<210>  10
<211>  9
<212>  PRT
<213>  Artificial

<220>
<223>  Oligonucleotide

<220>
<221>  MISC_FEATURE
<222>  (1)..(1)
<223>  Ac-NH-aaaaaaaa

<400>  10

Leu Tyr Ser Leu Tyr Ser Gly Leu Tyr
1               5


<210>  11
<211>  9
<212>  PRT
<213>  Artificial

<220>
<223>  Oligonucleotide


<220>
<221>  MISC_FEATURE
<222>  (1)..(1)
<223>  Ac-NH-aaaaaaaaa

<400>  11

Leu Tyr Ser Leu Tyr Ser Gly Leu Tyr
1               5


<210>  12
<211>  9
<212>  PRT
<213>  Artificial

<220>
<223>  Oligonucleotide


<220>
<221>  MISC_FEATURE
<222>  (1)..(1)
<223>  Ac-NH-aaaaaaaaaa

<400>  12

Leu Tyr Ser Leu Tyr Ser Gly Leu Tyr
1               5


<210>  13
<211>  12
<212>  PRT
<213>  Artificial

<220>

<223>  Oligonucleotide


<220>
<221>  MISC_FEATURE
<222>  (1)..(1)
<223>  Ac-NH-Lys(Dabcyl)-aaaaaaa

<400>  13

Leu Tyr Ser Leu Tyr Ser Gly Leu Tyr Gly Leu Tyr
1               5                   10


<210>  14
<211>  12
<212>  PRT
<213>  Artificial

<220>
<223>  Oligonucleotide


<220>
<221>  MISC_FEATURE
<222>  (1)..(1)
<223>  Ac-NH-Lys(Dabcyl)-aaaaaaa

<400>  14

Leu Tyr Ser Leu Tyr Ser Leu Tyr Ser Gly Leu Tyr
1               5                   10


<210>  15
<211>  23
<212>  DNA
<213>  Artificial

<220>
<223>  Oligonucleotide

<400>  15
tccaccttct ccaagaacta tat                                              23


<210>  16
<211>  23
<212>  DNA
<213>  Artificial

<220>
<223>  Oligonucleotide

<400>  16
tccaccttct caaagaacta tat                                              23

<210>  17
<211>  59
<212>  DNA
<213>  Artificial

<220>
<223>  Oligonucleotides


<220>
<221>  misc_feature
<222>  (1)..(1)
<223>  5' FAM

<220>
<221>  misc_feature
<222>  (2)..(2)
<223>  LNA

<220>
<221>  misc_feature
<222>  (4)..(4)
<223>  LNA

<220>
<221>  misc_feature
<222>  (6)..(6)
<223>  LNA

<220>
<221>  misc_feature
<222>  (8)..(8)
<223>  LNA

<220>
<221>  misc_feature
<222>  (10)..(10)
<223>  LNA

<220>
<221>  misc_feature
<222>  (12)..(12)
<223>  LNA

<220>
<221>  misc_feature
<222>  (13)..(13)
<223>  LNA

<220>
<221>  misc_feature
<222>  (15)..(15)
<223>  LNA

<220>
<221>  misc_feature
<222>  (17)..(17)
<223>  LNA

<220>
<221>  misc_feature
<222>  (59)..(59)
<223>  3' Dabcyl

<400>  17
ttctttcttt ttttttttat atagttcttt gagaaggtgg attttttttt ttctttctt      59


<210>  18
<211>  12
<212>  DNA
<213>  Artificial

<220>
<223>  Oligonucleotides


<220>
<221>  misc_feature
<222>  (1)..(1)
<223>  5' Dabcyl

<400>  18
aagaaagaaa aa                                                          12


<210>  19
<211>  13
<212>  DNA
<213>  Artificial

<220>
<223>  Oligonucleotide


<220>
<221>  misc_feature
<222>  (1)..(1)
<223>  5' Dabcyl

<400>  19
aagaaagaaa aaa                                                         13


<210>  20
<211>  14
<212>  DNA
<213>  Artificial

<220>
<223>  Oligonucleotide


<220>
<221>  misc_feature
<222>  (1)..(1)

<223>  5' Dabcyl

<400>  20
aagaaagaaa aaaa                                                          14


<210>  21
<211>  15
<212>  DNA
<213>  Artificial

<220>
<223>  Oligonucleotide


<220>
<221>  misc_feature
<222>  (1)..(1)
<223>  5' Dabcyl

<400>  21
aagaaagaaa aaaaa                                                         15


<210>  22
<211>  16
<212>  DNA
<213>  Artificial

<220>
<223>  Oligonucleotide


<220>
<221>  misc_feature
<222>  (1)..(1)
<223>  5' Dabcyl

<400>  22
aagaaagaaa aaaaaa                                                        16


<210>  23
<211>  17
<212>  DNA
<213>  Artificial

<220>
<223>  Oligonucleotide


<220>
<221>  misc_feature
<222>  (1)..(1)
<223>  5' Dabcyl

<400>  23
aagaaagaaa aaaaaaa                                                       17

<210>  24
<211>  18
<212>  DNA
<213>  Artificial

<220>
<223>  Oligonucleotide


<220>
<221>  misc_feature
<222>  (1)..(1)
<223>  5' Dabcyl

<400>  24
aagaaagaaa aaaaaaaa                                                    18

## Description

The invention relates to a detection molecule, in particular a detection probe for detecting a particular nucleic acid sequence, particularly for detecting a single nucleotide mismatch in a target nucleic acid.

The invention further relates to a method for modulating the conformational constraint of a probe as defined above for detecting a particular nucleic acid sequence, thereby improving its selectivity and sensitivity. Still further, the invention relates to the use of such a detection molecule for the detection of a nucleic acid sequence. The invention also relates to a kit for detecting a nucleic acid sequence.

### Background

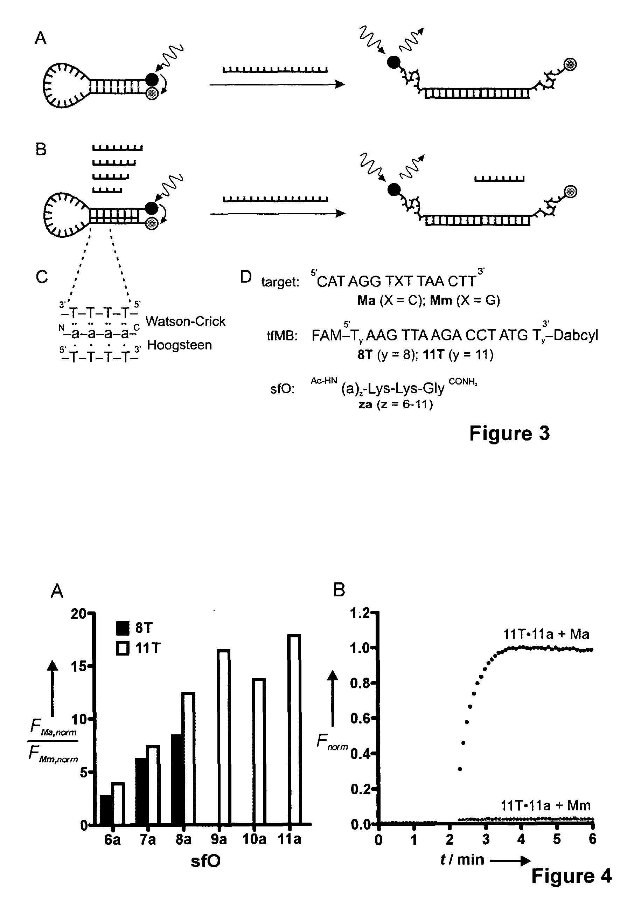

Detecting nucleic acid sequences is of central importance in modem life sciences. Given the rapid growth of the available sequence data, the diagnosis of DNA and RNA is likely to increase in significance over the next decade. The most useful methods employ probes that allow quantitative measurements in homogeneous solution, thereby omitting the need to separate bound from unbound probes (C. A. Foy, H. C. Parkes, Clin. Chem. 2001, 47, 990-1000 and R. T. Ranasinghe, T. Brown, Chem. Commun. 2005, 44, 5487-5502). Molecular beacons (MBs) (S. Tyagi, F. R. Kramer, Nat. Biotechnol. 1996, 14, 303-308; W. Tan, K. Wang, T. J. Drake, Curr. Op. Chem. Biol. 2004, 8, 547-553) have become a class of widely used probes for nucleic acid analysis. These probes carry a fluorophore and a quencher, generally at their termini. In absence of a complementary target, MBs form a stem-loop structure (Figure 3A), resulting in quenching of the fluorescence. Upon binding to a complementary target, the hairpin is opened, the labels are separated and fluorescence occurs. MBs have been used in quantitative polymerase chain reaction to monitor the formation of the amplicon in a sequence specific manner and in the imaging of pathogen RNA in living cells (S. Tyagi, F. R. Kramer, Nat. Biotechnol. 1996, 14, 303-308 and selected review: W. Tan, K. Wang, T. J. Drake, Curr. Op. Chem. Biol. 2004, 8, 547-553).

It is a useful feature that conformationally constrained oligonucleotide probes, such as MBs, recognize their targets with higher specificity than linear probes (G. Bonnet, S. Tyagi, A. Libchaber, F. R. Kramer, Proc. Natl. Acad. Sci. USA 1999, 96, 6171-6176). This property is desired in the analysis of single nucleotide alterations. The enhancement of specificity observed with structured probes implies that the selectivity of probe-target hybridization can be modulated by altering the degree of conformational constraint. For molecular beacons, this can be achieved by variation of the stem length and sequence. However, this modulation requires the synthesis of several different MBs. Among various conceptional modifications, replacements of the original DNA-Watson-Crick base paired stem by other pairing systems are rare examples. These include homo-DNA (C. Crey-Desbiolles, D. Ahn, C. Leumann, Nucleic Acids Res. 2005, 33, e77), LNA (L. Wang, C. J. Yang, C. D. Medley, S. A. Benner, W. Tan, J. Am. Chem. Soc. 2005, 127, 15664-15665) and G-quadruplex stems (A. Bourdoncle, A. Estevez Torres, C. Gosse, L. Lacroix, P. Vekhoff, T. Le Saux, L. Jullien, J.-L. Mergny, J. Am. Chem. Soc. 2006, 128, 11094-11105) as well as stemless peptide nucleic acid (PNA) molecular beacons (O. Seitz, Angew. Chem. 2000, 112, 3389-3392; Angew. Chem. Int. Ed. 2000, 39, 3249-3252; and H. Kuhn, V. V. Demidov, B. D. Gildea, J. M. Fiandaca, J. C. Coull, M. D. Frank-Kamenetskii, Antisense Nucleic Acid Drug Dev. 2001, 11, 265-270; and H. Kuhn, V. V. Demidov, J. C. Coull, J. M. Fiandaca, B. D. Gildea, M. D. Frank-Kamenetskii, J. Am. Chem. Soc. 2002, 124, 1097-1103). The cation dependent quadruplex stability was used to modulate the conformational constraint of the corresponding beacon (A. Bourdoncle, A. Estevez Torres, C. Gosse, L. Lacroix, P. Vekhoff, T. Le Saux, L. Jullien, J.-L. Mergny, J. Am. Chem. Soc. 2006, 128, 11094-11105), but in applications such as real-time PCR and in vivo measurements, the cation concentration can hardly be addressed.

Therefore, the problem underlying the present invention was to provide means that allow for the detection of a particular nucleic acid target sequence with high specificity or sensitivity.

### Description

This underlying problem is solved by a detection molecule according to the present invention. Such a detection molecule comprises an oligonucleotide and a probe. Said probe comprises a first stem forming portion with a fluorophore for generating a detection signal, a second stem forming portion with a quencher for quenching the detection signal from the fluorophore when the quencher is in proximity to the fluorophore, and a loop forming portion located between the first and the second stem forming portion, for allowing the first stem forming portion to form a stem structure together with the second stem forming portion of the probe and with the oligonucleotide. The oligonucleotide comprises a sequence portion that allows the formation of a triplex structure by interacting with the first and the second stem forming portions of the probe.

Detection of a particular nucleic acid sequence is achieved by sequence specific binding of the probe to a target nucleic acid, which can be DNA or RNA. Because of the enhanced properties of the detection molecule according to the present invention, it can successfully be used for detecting a single nucleotide polymorphism in a target nucleic acid.

In a preferred embodiment of the present invention, the oligonucleotide comprises a sequence that allows for the formation of base pairs with the first and the second stem forming portions of the probe. In particular, the oligonucleotide forms Watson-Crick and/or Hoogsteen base pairs with the first and/or second stem forming portions of the probe. Preferably, the oligonucleotide forms Watson-Crick base pairs with one stem forming portion, and Hoogsteen base pairs with the other stem forming portion of the probe.

In another preferred embodiment of the present invention, the oligonucleotide of the detection molecule comprises or consists of a peptide nucleic acid (PNA), in particular in that portion that forms base pairs with the probe. As will be shown in the examples, such a detection molecule is preferred over a detection molecule comprising a DNA oligonucleotide due to its higher sensitivity and/or selectivity.

It is also possible that the oligonucleotide comprises DNA, RNA, GNA (glycerol nucleic acid), PS-DNA (phosphorothioate DNA), L-DNA (enantiomeric DNA), OMe-RNA (2' O-methyl-RNA), MOE-RNA (2'-methoxyethoxy-RNA), NP (N3'-P5' phosphoramidates), FANA (2'-deoxy-2'-fluoro-β-D-arabinonucleic acid), LNA (locked nucleic acid), MF (morpholino phosphordiamidates), CeNA (cyclohexene nucleic acid), and/or tcDNA (tricyclo nucleic acid), all of which are known to a person of skill in the art.

Preferably, the probe comprises RNA, PNA, GNA, PS-DNA, L-DNA, OMe-RNA, MOE-RNA, NP, FANA, LNA, PNA, MF, CeNA, and/or tcDNA. Most preferably, the probe comprises or consists of DNA, in particular in that portion that forms base pairs with the oligonucleotide.

In order to generate a signal upon binding of the probe to a target nucleic acid, which is complementary in sequence to the sequence of a binding portion of the probe, the probe comprises a fluorophore for generating a signal. Various kinds of fluorophores are known in the art, including, but not limited to fluorescein isothiocyanate (FITC), tetramethylrhodamine isothiocyanate (TRITC), phycoerythrin, Cy7, fluorescein (FAM), Cy3, Cy3.5, Texas Red, LightCycler-Red 640, LightCycler Red 705, tetramethylrhodamine (TMR), rhodamine derivative (ROX), hexachlorofluorescein (HEX), Cy5, Cy5.5, rhodamine 6G (R6G), the rhodamine derivative JA133, Alexa Fluor 488, Alexa Fluor 546, Alexa Fluor 633, Alexa Fluor 555, Alexa Fluor 647, fluorescent nanoparticles, and fluorescent transition metal complexes, such as europium.

In order to prevent the generation of a signal when the probe is not bound to the target, the probe comprises a quencher, including, but not limited to 4-(4'-dimethyl-aminophenylazo)benzoic acid (Dabcyl), black hole quencher 1 (BHQ-1), black hole 25 quencher 2 (BHQ2), QSY-7, QSY-35, TRITC, Cy7, Cy3, Cy3.5, Texas Red, LightCycler-Red 640, LightCycler Red 705, TMR, ROX, HEX, Cy5, Cy5.5, the rhodamine derivative JA133, Alexa Fluor 546, Alexa Fluor 633, Alexa Fluor 647). Upon binding of the probe to the target, fluorophore and quencher are being separated and a signal is generated which can be detected. The strength of this signal allows a conclusion about the amount and/or number of the target sequence present in a given sample, in particular when compared to a standard.

In one preferred embodiment, the first and the second stem forming portions of the probe comprise a poly(T) sequence and the oligonucleotide binding to them comprises a poly(a) sequence.

In order to enhance quenching of the fluorophore while the detection molecule is not bound to a target, and therefore to reduce the background signal, the oligonucleotide comprises a quencher group at at least one end of the oligonucleotide. In a preferred embodiment of the invention, an oligonucleotide is used with a quencher at either end, particularly when the oligonucleotide can bind to the stem forming portions of the probe in a non oriented fashion. Such oligonucleotides are also being referred to as "superquenched" herein.

In another preferred embodiment of the present invention, the oligonucleotide further comprises a functional moiety, such as an additional fluorophore or a quencher, including, but not limited to the examples listed above, and/or a handle for immobilization, including, but not limited to an additional oligonucleotide sequence, a free thiol function, biotin, avidin, an azide or alkyne, a thioester, cysteine as well as an antibody or a hapten to an antibody.

Preferably, the oligonucleotide further comprises a chemically or biologically active moiety that triggers a signal upon release of the oligonucleotide from the probe. Such a moiety includes, but is not limited to, a recognition site for a restriction enzyme. Therefore, when the oligonucleotide is not bound to the probe anymore due to the presence of a nucleic acid with a target sequence for the probe, the oligonucleotide is cleaved in the presence of a suitable restriction enzyme. The cleavage of the oligonucleotide can then be detected.

Additionally, the active moiety can also be an mRNA sequence that is part of the oligonucleotide. Upon release of the oligonucleotide from the probe, the mRNA can be translated to produce a detectable protein. In another embodiment, the moiety is an antibody recognition site or an enzyme recognition site, which allows for a detectable binding and/or a detectable reaction to occur.

It is advantageous that the oligonucleotide has the length of the stem forming portion of the probe. Alternatively, the oligonucleotide can also be shorter than the length of the stem forming portion.

In another embodiment of the invention, the oligonucleotide is longer than the stem forming portion of the probe. Thereby, a portion of the oligonucleotide forms an overhang that does not form base pairs with the probe. This overhang of the oligonucleotide can be used to immobilize the oligonucleotide, and therefore the detection molecule on a solid support, such as a chip. This chip can than be used in a spot assay, which is known in the art.

In order to allow for orientation specific binding of the oligonucleotide and the stem forming portions of the probe to each other in a triplex, the first and the second stem forming portions of the probe each contain at least one base for oriented binding of the oligonucleotide, and the oligonucleotide contains at least one base for oriented binding to the probe. Thereby, said base(s) for oriented binding of the probe interact such with said base(s) for oriented binding of the oligonucleotide, that the orientation and binding position of the oligonucleotide to the probe is unequivocally determined.

In a preferred embodiment, the oriented oligonucleotide comprises a fluorphor, namely at that end that is located adjacent to the quencher of the probe in the detection molecule. Thereby, upon binding of the probe to a target sequence, an additional signal is generated stemming from the fluorpohor of the oligonucleotide. Thereby, the signal strength is enhanced.

Alternatively, it is also possible to use a probe without a quencher if the fluorphor of the probe and the fluorphor of the oligonucleotide are oriented such to each other in a triplex, that the fluorophors quench each other.

The underlying problem is also solved by a method for modulating the conformational constraint of a probe, and thereby enhancing its specificity and/or sensitivity.

The probe is one with the same properties as described above. Specifically, it comprises a first stem forming portion with a fluorophore for generating a detection signal, a second stem forming portion with a quencher for quenching the detection signal from the fluorophore when the quencher is in proximity to the fluorophore, whereby the first stem forming portion can form a stem structure with the second stem forming portion, and a loop forming portion located between the first and the second stem forming portion for allowing the first stem forming portion to form a stem structure with the second stem forming portion.

According to the invention, an oligonucleotide is provided that comprises a sequence portion that allows it to form a triplex structure with the first and second stem forming portions of the probe. Thereby, the sensitivity and selectivity of any given probe can be improved by choosing the appropriate oligonucleotide length. This method allows for "tuning" of both specificity and sensitivity of DNA-detection without resynthesis of the dual labeled probe, thereby saving costs and time.

An optimized probe with enhanced sensitivity and/or selectivity is achieved in particular by providing oligonucleotides of varying length that form a triplex with the given probe.

Preferably, the melting temperature of the detection molecule formed by interaction of such oligonucleotides of varying length with the probe is determined to optimize sensitivity and/or selectivity of the probe regarding the detection of a given nucleic acid sequence, in particular regarding the detection of a single nucleotide polymorphism in a target nucleic acid.

The detection molecule, in particular the oligonucleotide used in the method according to the present invention can exhibit any feature described above.

The underlying problem is also solved by using a detection molecule as defined above for the quantitative detection of a nucleic acid sequence, in particular for detection of a single nucleotide polymorphism in a target nucleic acid.

Preferably, the detection molecule is incubated with a target sequence and the signal generated upon binding of the probe through separation of the quencher and the fluorophore is detected. By comparison of the signal strength to a standard, the amount of target sequence can be determined.

The detection molecule according to the present invention is preferably used for the detection of single nucleotide polymorphisms, in particular those that are related to a disease of a patient. Thereby, the present method can be used for diagnosis and/or prediction of a genetic-based disease.

The underlying problem is also solved by a kit for detecting a nucleic acid sequence, in particular for detecting a single nucleotide polymorphism in a target nucleic acid. Such a kit comprises an oligonucleotide and a probe. The probe comprises a first stem forming portion with a fluorophore for generating a detection signal, a second stem forming portion with a quencher for quenching the detection signal from the fluorophore when the quencher is in proximity to the fluorophore, and a loop forming portion located between the first and the second stem forming portion, for allowing the first stem forming portion to form a stem structure together with the second stem forming portion of the probe and with the oligonucleotide, whereby the oligonucleotide comprises a portion that allows it to form a triplex structure with the first and second stem forming portions of the probe.

For preferred embodiments of the oligonucleotide and the probe of the kit, reference is made to the description above.

The kit may comprise further components, in particular components needed for performing a quantitative polymerase chain reaction, such as buffers, primers, dNTPs and a (heat stable) polymerase.

The underlying problem is also solved by an oligonucleotide or a probe according to SEQ ID NOs 1 to 24, which are further described below with reference to the examples.

### Brief description of the figures

- **Figure 1:**: Denaturation curves of tfMB 11T (SEQ ID NO 4) with the sfO 7a (SEQ ID NO 8): (A) crude data, (B) corrected denaturation curves and (C) after normalization for the calculation of the selectivity.
- **Figure 2:**: Denaturation curves of tfMB 8T (SEQ ID NO 3) with the sfO 7a (SEQ ID NO 8): (A) crude data, (B) corrected denaturation curves and (C) after normalization for the calculation of the selectivity.
- **Figure 3:**: Comparison of molecular beacon (MB, A) and triplex molecular beacon (tMB, B) for the detection of complementary DNA-oligonucleotides. (C) Poly(a)·2poly(T) structure, forming the triplex stem region. (D) Sequences of DNA (target (SEQ ID NOs 5 and 6), tfMB (SEQ ID NOs 3 and 4)) and PNA (sfO) (SEQ ID NOs 7 to 12).
- **Figure 4:**: (A) Maximal normalized selectivities of both tfMBs with different sfOs calculated from thermal denaturation experiments (see figures 1 and 2). (B) Kinetics monitored at λₑₓ = 492 nm and λₑₘ = 525 nm at 43 °C. Addition of Ma (SEQ ID NO 5) or Mm (SEQ ID NO 6) after 2 min. Conditions: 0.2 µmol/l 11T, 0.3 µmol/l 11a and 0.4 µmol/l target (Ma (SEQ ID NO 5) or Mm (SEQ ID NO 6)); buffer: 10 mmol/l Tris·HCl, 30 mmol/l KCl, 2 mmol/l MgCl₂, pH 9.
- **Figure 5:**: (A) Fluorescence increases of both tfMBs with different sfOs at 20 °C calculated from thermal denaturation experiments (see figures 1 and 2). (B) Kinetics monitored at λₑₓ = 492 nm and λₑₘ = 525 nm at 20 °C. Conditions: 0.2 µmol/l tfMB (8T (SEQ ID NO 3) or 11T (SEQ ID NO 4)), 0.25 µmol/l 7a (SEQ ID NO 8) and 0.6 µmol/l Ma (SEQ ID NO 5); buffer: 20 mmol/l NaH₂PO₄, 100 mmol/l KCl, 2 mmol/l MgCl₂, pH 9.0.
- **Figure 6:**: (A) "Superquenched" triplex molecular beacons (Sequences: 7a** ^{AcHN}Lys(Dabcyl)-a₇-Lys(Dabcyl)-Lys₂-Gly^{CONH}2 (SEQ ID NO 14), 7a* ^{AcHN}Lys(Dabcyl)-a₇-Lys₂-Gly₂^{CONH}2 (SEQ ID NO 13)). (B) Kinetics monitored at λₑₓ = 492 nm and λₑₘ = 525 nm at 20 °C. Conditions: 0.2 µmol/l 11T (SEQ ID NO 4), 0.25 µmol/l sfO (7a (SEQ ID NO 8), 7a* (SEQ ID NO 13) or 7a**(SEQ ID NO 14)) and 0.6 µmol/l Ma (SEQ ID NO 5); buffer: 10 mmol/l Tris·HCl, 30 mmol/l KCl, 2 mmol/l MgCl₂, pH 9.
- **Figure 7:**: Oriented triplex molecular beacons (G-C base pairs in grey, marked by an arrow).
- **Figure 8:**: Melting temperatures in °C. Conditions: 1 µM oligonucleotides; buffer: 10 mmol/l Tris·HCl, 30 mmol/l KCl, 2 mmol/l MgCl₂, pH 9.
- **Figure 9:**: Detection molecules according to the present invention (triplex Molecluar Beacons (tMB)).

### Examples

The following examples are described with reference to the figures.

In the embodiments described in the examples, the oligonucleotide according to the invention is also referred to as the stem forming oligonucleotide (sfO), the probe according to the invention is also referred to as a triplex forming Molecular Beacon (tfMB), and the detection molecule according to the invention, comprising both probe and oligonucleotide, is also referred to as a triplex Molecular Beacon (tfMB).

### Materials and instruments

The PNA monomer Fmoc-a(Bhoc)-OH was purchased from Applied Biosystems. TCTU was obtained from Iris Biotech. Novasyn TGR resin, protected amino acids and Fmoc-Lys(Dabcyl)-OH were purchased from Novabiochem. DNA was purchased from BioTeZ Berlin-Buch GmbH, Germany, (non-labeled salt free and labeled in HPLC quality). Dry DMF (H₂O < 0.01 %) was purchased from Fluka. Water was purified with a Milli-Q ultra pure water purification system. Automated linear solid-phase synthesis of non-labeled PNA was performed by using an Intavis ResPep parallel synthesizer equipped with micro scale columns for PNA synthesis essentially as previously described (T. N. Grossmann, O. Seitz, J. Am. Chem. Soc. 2006, 128, 15596-15597). The same protocol was used for the synthesis of Dabcyl-Iabeled PNA.

### Determination of melting temperatures (T_{M})

UV melting curves were measured at 260 nm by using a Varian Cary 100 spectrometer (UV quartz cuvettes: 4x 10 mm) equipped with a peltier block. A degassed aqueous solution (10 mmol/l Tris-HCI, 30 mmol/l KCl, 2 mmol/l MgCl₂, pH 9) was used as buffer. The oligonucleotides were mixed at 1:1 ratio, and the solutions were adjusted to a final duplex/triplex concentration of 1 µmol/l. Prior analysis, the samples were heated to the maximum temperature of 85 °C and cooled to starting temperature of 15 °C. Melting curves were recorded at a rate of 0.5 °C/min. T*_{M}* values were defined as the maximum of the first derivative of the melting curve.

non-labeled tfMB: ⁵'T_{y} AAG TTA AGA CCT ATG T_{y}^{3'}
8T-nl (y = 8) (SEQ ID NO 1); 11T-nl (y = 11) (SEQ ID NO 2)

### Fluorescence denaturation experiments

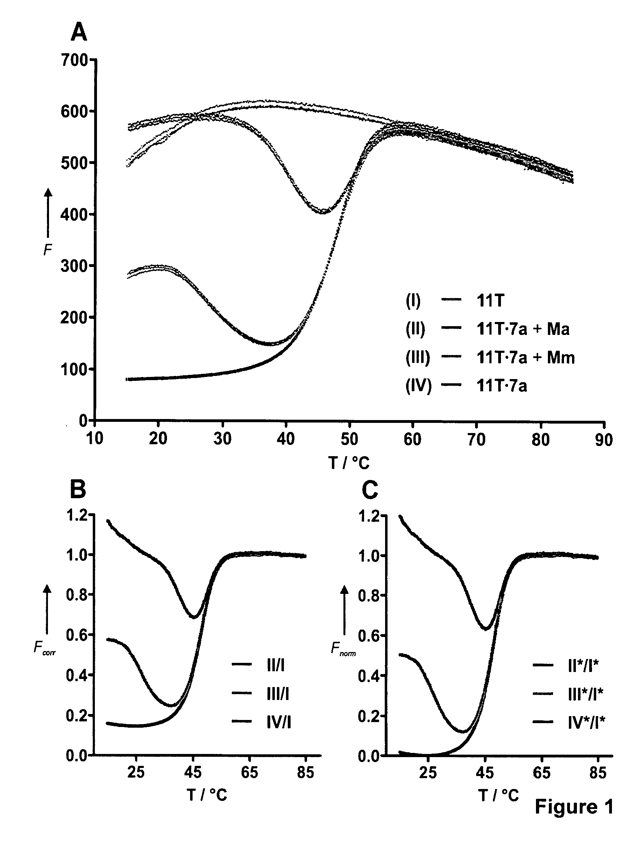

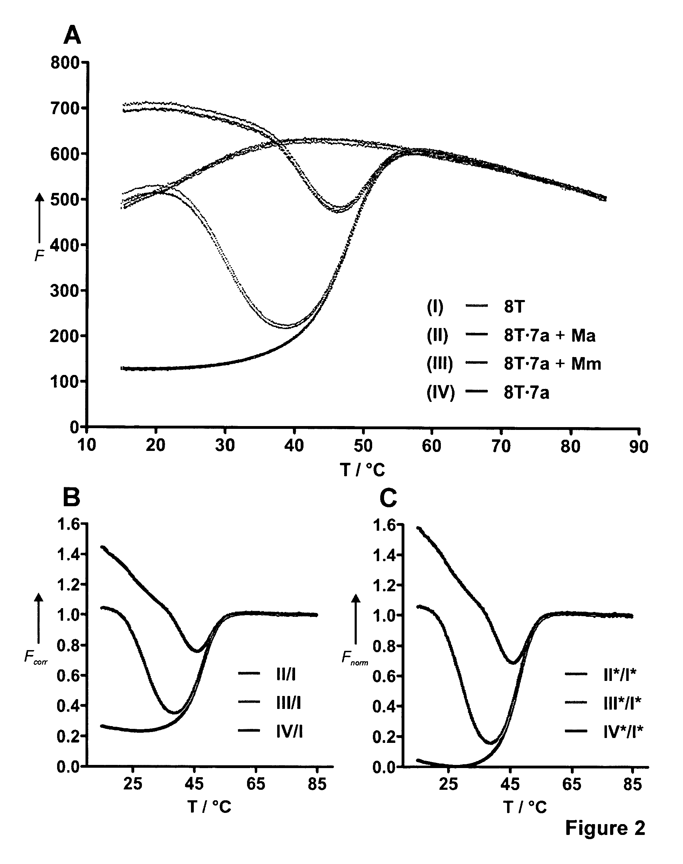

Fluorescence melting curves were measured using a Varian Cary Eclipse spectrometer (fluorescence quartz cuvettes: 4x 10 mm; settings: λₑₓ = 485 nm (slit 5 nm), λₑₘ = 525 nm (slit 10 nm)) equipped with a peltier block. A degassed aqueous solution (10 mmol/l Tris·HCl, 30 mmol/l KCl, 2 mmol/l MgCl₂, pH 9) was used as buffer. The oligonucleotides were used at the following concentrations: 0.2 µmol/l tfMB (8T (SEQ ID NO 3) and 11T (SEQ ID NO 4)), 0.3 µmol/l sfO (6a - 11a (SEQ ID NOs 7 to 12)) and 0.4 µmol/l target (Ma (SEQ ID NO 5) and Mm (SEQ ID NO 6)). Prior to analysis, the samples were heated to the maximum temperature of 85 °C and cooled to a starting temperature of 15 °C. Melting curves were recorded in triplicates at a rate of 0.75 °C/min at a data interval of 0.1 °C. Hybridization of the triplex molecular beacons is highly reversible and no hystereis could be observed (see Figure 1/2.A). Prior to analysis, two calibrations have been performed: First, the mean value of the three denaturation curves was calculated and adjusted to yield the same mean value in the interval of 78 °C - 80 °C, where all beacons are fully denatured (to correct for small pipetting variations). Secondly, all curves were divided by the fluorescence of the tfMB without stem forming oligonucleotide (to correct for the thermal dependence of the fluorescence intensity). Figures 1 and 2 show the corresponding curves before and after calibration for the combinations 8T·7a (SEQ ID NOs 3 and 8) and 11T·7a (SEQ ID NOs 4 and 8).

For the determination of the maximal selectivity, all curves have been normalized by setting the minimum value of the denaturation curve of tfMB + sfO to zero. From the normalized denaturation curves (Figure 1/2.C) we have calculated the selectivities at each temperature as *F*_{*II**/*I*, T*} /*F*_{*IV**/*I**, *T*} where *F*_{*II**/*I*, T*} and *F*_{*IV**/*I*, T*} are the normalized fluorescence intensities for the Ma and Mm case at the same temperature (T). The maximum of the normalized selectivity for each combination is given in Table 1 together with the corresponding temperature. As expected, both the selectivities and the corresponding temperatures increase with the length of the stem forming oligonucleotide until in case of the tfMB 11T (SEQ ID NO 4) reaching a plateau for the 9a - 11a sfOs (SEQ ID NOs 10 to 12).

Fluorescence increases at 20 °C have been calculated from the corrected denaturation curves (Figure 1/2.B) as *F*_{*II*/*I*, *20°C*} / *F*_{*III*/*I, 20°C*}. The combined results are given in Table 2. In this case, there are two opposing effects that determine the fluorescence enhancement. If the stem forming oligonucleotides are shorter, the quenching without target can be less effective due to the lower stability and higher flexibility which decreases the possible fluorescence enhancement. On the other hand, elongation of the stem forming oligonucleotide will hinder opening which reduces the fluorescence increase as well.

**Table 1. Maximal normalized selectivities calculated from the thermal denaturation curves as stated above.**

| **Maximum normalized selectivity** | | |
|---|---|---|
| **sfO** | **8T (at T / °C)** | **11T (at T / °C)** |
| **6a** | 2.7 (36.3) | 3.9 (33.2) |
| **7a** | 6.2 (38.2) | 7.4 (36.7) |
| **8a** | 8.4 (39.0) | 12.4 (39.3) |
| **9a** | - | 16.4 (42.2) |
| **10a** | - | 13.4 (42.4) |
| **11a** | - | 17.2 (42.4) |

**Table 2. Fluorescence increases at 20 °C calculated from the thermal denaturation curves.**

| **Fluorescence increase at 20 °C** | | |
|---|---|---|
| **sfO** | **8T** | **11T** |
| **6a** | 5.6 | 6.3 |
| **7a** | 5.5 | 7.3 |
| **8a** | 5.2 | 6.1 |
| **9a** | - | 4.2 |
| **10a** | - | 3.6 |
| **11a** | - | 3.7 |

### Kinetic experiments

Kinetic experiments were measured using a Varian Cary Eclipse spectrometer (fluorescence quartz cuvettes: 4x10 mm) equipped with a peltier block. All measurements were carried out in a buffered solution (10 mmol/l Tris-HCI, 30 mmol/l KCI, 2 mmol/l MgCl₂, pH 9). Time courses of FAM fluorescence were measured using the following settings: λₑₓ = 485 nm (slit 5 nm), λₑₘ = 525 nm (slit 10 nm). Oligonucleotides were diluted in H₂O to approx. 250 µmol/l to serve as stock solutions. To the buffer (final volume 1 ml) the appropriate amount of tfMB (8T (SEQ ID NO 3) or 11T (SEQ ID NO 4)) was added followed by the addition of sfO (6a - 11a, (SEQ ID NOs 7 to 12)). After fluorescence intensity reached its minimum, the time was reset to zero and after 2 min data acquisition was paused (for approx. 45 sec.) and the required amount of target (Ma (SEQ ID NO 5) and Mm (SEQ ID NO 6)) was added to the cuvette. After mixing the solution by turning the cuvette for two times, data acquisition was continued.

### Figure 3:

The inventors of the present invention have surprisingly found that triplex-based molecular beacons (tMBs, Figure 3B) enable a precise modulation of the conformational constraint by using a single probe (triplex forming Molecular Beacon, tfMB) labeled with a fluorophore and a quencher in combination with several stem forming oligonucleotides (sfOs). In this setup, only one dual labeled probe is needed and it is the sfO that allows modulation of the selectivity and sensitivity of the tMB towards target DNA-sequences. Furthermore, the modular assembly facilitates the additional incorporation of functionalities that can be easily introduced via the sfO. "Superquenched" tMBs were prepared by appending additional quencher moieties to the sfO.

The tMB of the example shown is designed in a way that the stem region is formed by a poly(a)·2poly(T) structure (Figure 3C) (M. D. Frank-Kamenetskii, S. M. Mirkin, Annu. Rev. Biochem. 1995, 64, 65-95). The sfO oligo(a) segment is composed of PNA, a DNA analogue bearing an uncharged *N*-(2-aminoethyl)glycine backbone. PNA is capable of forming more stable duplexes and triplexes with complementary DNA than corresponding DNA (P. Nielsen, M. Egholm, R. Berg, O. Buchardt, Science 1991, 254, 1497-1500; P. E. Nielsen, Curr. Opin. Biotechnol. 2001, 12, 16-20). The sfO was designed to bind two poly(T) arm segments of the tfMB via Watson-Crick and Hoogsteen base pairing, respectively.

Two tfMB systems (8T (SEQ ID NO 3) and 11T (SEQ ID NO 4), Figure 3D) were investigated. Both tfMBs consist of a central DNA-sequence complementary to the matched target-sequence (Ma (SEQ ID NO 5)). This central sequence is flanked by poly(T) regions labeled with 6-FAM/Dabcyl as fluorphor/quencher. The sfO lengths varied from 6 to 11 adenine residues (SEQ ID NOs 7 to 12). The stability of duplexes formed between tfMB and target (tfMB·target) in absence of sfOs, as well as the stability of triplexes (tfMB•sfO) in absence of target were studied in initial UV denaturation experiments.

Table 3 shows the melting temperatures (T*_{M}*) of the non-labeled tfMBs (8T-nl (SEQ ID NO 1) and 11T-nl (SEQ ID NO 2)) complexed with targets or sfOs. The expected lower stability (ΔT*_{M}* = -11 °C) of the single-base mismatched duplexes tfMB·Mm compared to the perfectly matched duplexes tfMB·Ma was observed. Melting temperatures of tfMB•sfO triplexes increased with increasing sfO length. This allows the precise modulation of the conformational constraint of the tMB.

**Table 3. Melting temperatures (in °C) of non-labeled triplex forming molecular beacons (tfMBs) in complex with perfect complementary target (Ma (SEQ ID NO 5)), single mismatched target (Mm (SEQ ID NO 6)) and with the different stem forming oligonucleotides (sfO) (SEQ ID NOs 1 and 2). Conditions: 1 µmol/l oligonucleotides; buffer: 10 mmol/l Tris·HCl, 30 mmol/l KCl, 2 mmol/l MgCl₂, pH 9.**

| | **tfMB•Target** | | **tfMB•sfO** | | | | | |
|---|---|---|---|---|---|---|---|---|
| | **Ma** | **Mm** | **6a** | **7a** | **8a** | **9a** | **10a** | **11a** |
| **8T-nl** | 47 | 36 | 38 | 44 | 49 | - | - | - |
| **11T-nl** | 48 | 37 | 44 | 48 | 53 | 57 | 59 | 60 |

### Figure 4:

To compare the ability of each tfMB·sfO triplex beacon to discriminate between matched (Ma (SEQ ID NO 5)) and single mismatched (Mm (SEQ ID NO 6)) target, denaturation experiments were performed monitored by FAM-fluorescence in absence of target, and in presence of matched (Ma) and mismatched (Mm) target. Denaturation curves were reversible and no hystereis was observed (see above). To correct for small pipetting variations and the intrinsic temperature dependence of fluorescence, normalized match/mismatch selectivities were calculated (see above). Figure 4A shows the highest normalized match/mismatch selectivity determined for each tfMB•sf0 complex. The selectivity improved with increasing triplex stability as higher selectivities were achieved with 11T than with 8T, and with longer sfOs. For 11T, a plateau in terms of selectivity was reached at an sfO length of nine adenine bases (SEQ ID NO 10). The maximal normalized selectivities of 11T with 9a (SEQ ID NO 10) (16.4), 10a (SEQ ID NO 11) (13.4) and 11 a (SEQ ID NO 12) (17.2) were observed at temperatures between 42 °C and 43 °C.

In addition, real-time kinetic measurements were performed at 43 °C with the most selective tMB, 11T·11a. Figure 4B gives the time course of normalized FAM-fluorescence before and after addition of two equivalents of target DNA. Whereas in presence of mismatched target (Mm, light grey) almost no signal increase occurred, a significant increase in presence of matched target (Ma, dark grey) was observed.

### Figure 5:

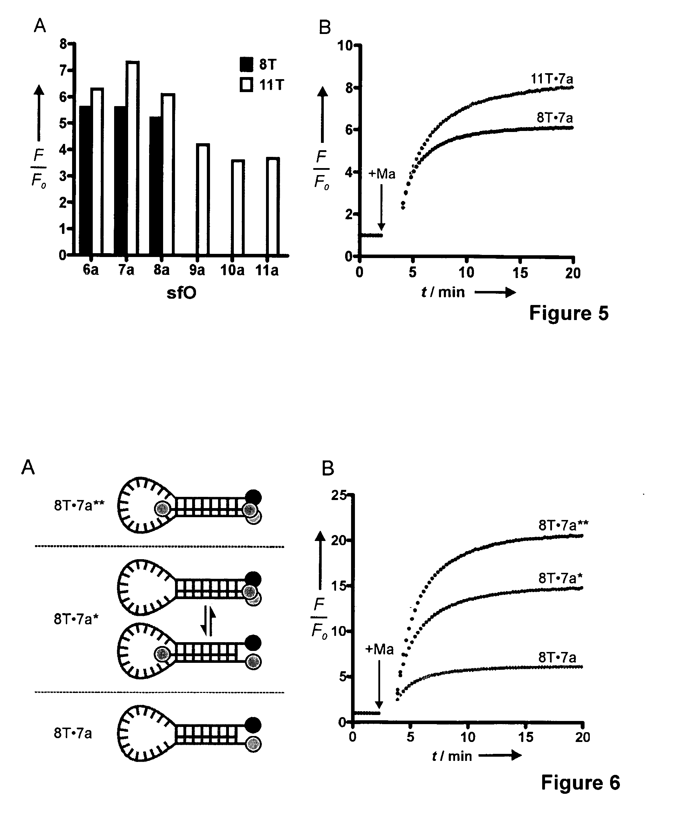

The next aim was to investigate the sfO length dependence of the fluorescence increase upon addition of matched target (Ma (SEQ ID NO 5)). From the denaturation experiments described above, the fluorescence increases at 20 °C was calculated (Figure 5A). In agreement with an increasing restoring force upon triplex elongation, going from 7a (SEQ ID NO 8) to 11a (SEQ ID NO 12), a lower tendency for opening of the tMB indicated by a reduced fluorescence increase was observed. A maximum in the fluorescence increase was reached with sfO 7a (SEQ ID NO 8), presumably, due to the incapability of the shorter sfO 6a (SEQ ID NO 7) to close the tMB quantitatively. The long-stem triplex beacons 11T (SEQ ID NO 4) gave higher fluorescence intensification than the shorter-stem beacons 8T (SEQ ID NO 3). This is due to more efficient fluorescence quenching in 11T•sfO.

Figure 5B shows real-time measurements of the addition of three equivalents Ma to 8T•7a and 11T•7a, respectively. Under these conditions, a 6-fold and 8-fold fluorescence increase was achieved. Notably, fluorescence signaling occurred even with the most stable tMB (11T·11a). This is surprising when considering the high stability of the triplex stem (T*_{M}* = 60 °C, Table 1) and the comparably low stability of the 11T·target complex (T*_{M}* = 48 °C). It can be speculated that hybridization of the target first disrupts Hoogsteen interactions.

The affinity of the long sfO 11a (SEQ ID NO 12) for the Watson-Crick strand of 11T (SEQ ID NO 4) may be sufficient to keep both target Ma (SEQ ID NO 5) and 11a (SEQ ID NO 12) complexed to 11T (SEQ ID NO 4). In contrast, the shorter sfO 7a (SEQ ID NO 8) should have a higher tendency for dissociation.

### Figure 6:

The modular assembly of triplex molecular beacons enables a very facile introduction of further functionalities. As an example, "superquenched" tMBs were constructed by attaching additional Dabcyl-quencher moieties to sfO 7a (SEQ ID NO 8) (7a* (SEQ ID NO 13) and 7a** (SEQ ID NO 14), Figure 6A). It was the objective to increase the fluorescence enhancement upon target addition by reducing the fluorescence of the closed tMB. Indeed, the use of the Dabcyl-sfOs 7a*(SEQ ID NO 13) and 7a** (SEQ ID NO 14) resulted in dramatic increases of the quenching efficiency, from 77.6 % (8T·7a) to 98.3 % (8T·7a**) and from 86.0 % (11T·7a) to 96.9 % (11T·7a**). Real-time measurements of the 8T (SEQ ID NO 3) beacon system with Dabcyl-Iabeled sfOs (SEQ ID NOs 8, 13, and 14) (Figure 6B) revealed significantly enlarged fluorescence increases upon hybridization to Ma (7a: 6.2-fold, 7a*: 14.9-fold, 7a**: 20.8-fold).

In summary, triplex molecular beacons are able to report the presence of DNA targets in homogeneous solution by enhancements of fluorescence signals. The addition of target induced up to 8-fold fluorescence enhancements when using non-labeled sfOs, and up to 21-fold enhancements when using Dabcyl-Iabeled sfOs. The use of improved fluorophore/quencher pairs leads to even higher signal increases. The obtained specificity of fluorescence signaling as far as match/mismatch discrimination is concerned, compares well with the specificity achieved with conventional duplex molecular beacons.

The central hallmark of triplex molecular beacons is the feasibility to adjust the specificity and the sensitivity of a given dual labeled oligonucleotide by choosing the appropriate stem forming oligonucleotide. This property is of advantage in optimization studies, for example in real-time quantitative PCR. In contrast, optimization of alternative DNA detection probes usually requires resynthesis (D. J. French, C. L. Archard, T. Brown, D. G. McDowell Mol. Cell. Probes 2001, 15, 363-374; and N. Svanvik, A. Stahlberg, U. Sehlstedt, R. Sjoback, M. Kubista Anal. Biochem. 2000, 287, 179-182; and O. Köhler, O. Seitz, Chem. Commun. 2003, 2938-2939; and O. Köhler, D. V. Jarikote, O. Seitz ChemBioChem 2005, 6, 69-77). The modular assembly of tfMB and sfO allows for a very facile introduction of further functionalities without increasing the complexity and cost of the beacon synthesis.

The attachment of fluorescence quenchers to the sfO was demonstrated, which resulted in increases of fluorescence signaling. Other conjugation opportunities, such as fluorphores, handles for immobilization etc. are possible. The extension with chemically or biologically active moieties that trigger a signal upon release of the sfO will add to the versatility of triplex molecular beacons as a new class of easily tunable DNA detection probes.

Triplex-formation is used to construct a stem-loop probe, which is opened upon binding of the DNA target. The triplex molecular beacon comprised a single DNA (dark gray), labeled with a fluorophore and a quencher, and a stem forming oligomer (black). Both specificity and sensitivity of DNA-detection can be modulated without resynthesis of the dual labeled beacon. The modular concept also facilitates the introduction of further functionalities such as additional quenchers in the assembly of "superquenched" beacons.

### Figure 7:

When a poly(A/a)·2poly(T) stem is used, it does not allow to control the orientation of the sfO neither in the closed nor in the open state of the probe. This complicates the design of tMBs with additional functionalities in the sfOs.

To alleviate this problem, two G-C base pairs are introduced that allow the precise orientation of the sfO in the closed as well as the open state of the probe. Therefore, one can predict the relative orientation of tfMB and sfO labels (compare Figure 5A (8T·7a*) and 7).

Also, the modulation of the stability by varying the sfO length affects the quenching efficiency in the closed state. Therefore, only a limited range of sfO lengths can be used with one tfMB.

The introduction of two G-C base pairs allows the fixation of the sfO at the open end of the hairpin structure. In conclusion, the quenching efficiency by closing the beacon is not affected by the length of the sfO.

### Figure 8:

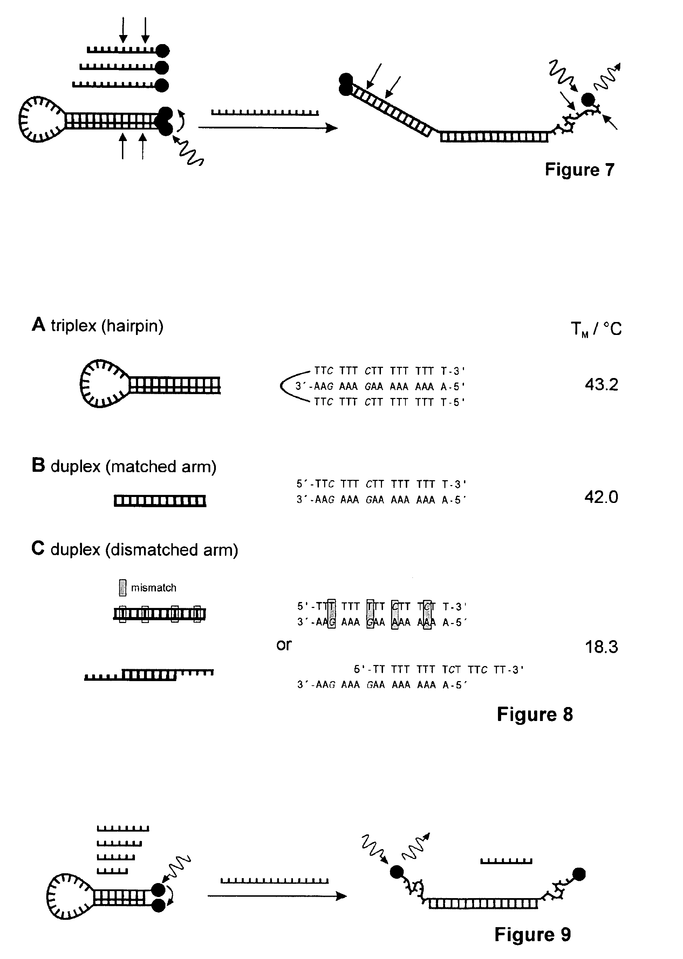

The melting temperatures of the matched and mismatched arm segments in complex with the sfOs (Figure 8B and C) underline the possibility to achieve the desired orientation of the sfO in the open state and suggest the same for the closed state.

The present invention is illustrated in a proposed detection of a mutation in the cystic fibrosis transmembrane conductance regulator (CFTR) gene.
wild type target (antisense)
5'-TCC ACC TTC TCC AAG AAC TAT AT-3' (SEQ ID NO 15)
mutant target (antisense)
5'-TCC ACC TTC TCA AAG AAC TAT AT-3'(SEQ ID NO 16)

Compared to DNA, LNA-modified oligonucleotides exhibit an increased stability in the formation of Hoogsteen base pairs. Therefore, an LNA-modified tfMB arm segment can be applied to increase the stability of the tMB stem region.
triplex forming Molecular Beacon (tfMB), underlined nucleobases are LNAs 
5’ FAM-TTC TTT CTT TTT TTT TTT ATA TAG TTC TTT GAG AAG GTG GA TTT
TTT TTT TTC TTT CTT-Dabcyl 3’ (SEQ ID NO 17)
 stem forming Oligos (sfOs)
5' Dabcyl-AAG AAA GAA (A)ₙ₋3' (n = 3-9) (SEQ ID NOs 18-24)

### Figure 9:

Triplex-formation is used to construct a stem-loop probe, which is opened upon binding of the DNA target. The triplex molecular beacon comprised a single DNA (dark gray), labeled with a fluorophore and a quencher, and a stem forming oligonucleotide (black). Both specificity and sensitivity of DNA-detection can be modulated without resynthesis of the dual labeled beacon. The modular concept also facilitates the introduction of further functionalities, such as additional quenchers in the assembly of "superquenched" beacons.

## Claims

1. A detection molecule, in particular a detection probe for detecting a nucleic acid sequence, in particular for detecting a single nucleotide polymorphism in a target nucleic acid, comprising
a) an oligonucleotide, and
b) a probe, comprising
- a first stem forming portion with a fluorophore for generating a detection signal,
- a second stem forming portion with a quencher for quenching the detection signal from the fluorophore when the quencher is in proximity to the fluorophore, and
- a loop forming portion located between the first and the second stem forming portion, for allowing the first stem forming portion to form a stem structure together with the second stem forming portion of the probe and with the oligonucleotide,
whereby the oligonucleotide comprises a portion that allows it to form a triplex structure with the first and second stem forming portions of the probe.

2. The detection molecule according to claim 1, wherein the oligonucleotide comprises DNA, RNA, GNA, PS-DNA, L-DNA, OMe-RNA, MOE-RNA, NP, FANA, LNA, MF, CeNA, and/or tcDNA, preferably peptide nucleic acid (PNA).

3. The detection molecule according to claim 1 or 2, wherein the probe comprises RNA, PNA, GNA, PS-DNA, L-DNA, OMe-RNA, MOE-RNA, NP, FANA, LNA, MF, CeNA, and/or tcDNA, preferably DNA.

4. The detection molecule according to any of the preceding claims, wherein the oligonucleotide comprises a sequence that allows for the formation of base pairs with the first and the second stem forming portions of the probe.

5. The detection molecule according to any of the preceding claims, wherein the oligonucleotide forms Watson-Crick and/or Hoogsteen base pairs with the first and/or second stem forming portions of the probe.

6. The detection molecule according to any of the preceding claims, wherein the oligonucleotide comprises a group for quenching the detection signal from the fluorophore of the probe at at least one end of the oligonucleotide.

7. The detection molecule according to claim 6, wherein the group is Dabcyl, BHQ-1, BHQ-2, QSY-7, QSY-35, TRITC, Cy7, Cy3, Cy3.5, Texas Red, LightCycler-Red 640, LightCycler Red 705, TMR, ROX, HEX, Cy5, Cy5.5, the rhodamine derivative JA133, Alexa Fluor 546, Alexa Fluor 633, or Alexa Fluor 647.

8. The detection molecule according to any of the preceding claims, wherein the oligonucleotide further comprises a functional moiety, such as a fluorophore like FITC, TRITC, phycoerythrin, Cy7, FAM, Cy3, Cy3.5, Texas Red, LightCycler-Red 640, LightCycler Red 705, TMR, ROX, HEX, Cy5, Cy5.5, R6G, JA133, Alexa Fluor 488, Alexa Fluor 546, Alexa Fluor 633, Alexa Fluor 555, Alexa Fluor 647, fluorescent nanoparticles, fluorescent transition metal complexes, such as europium.

9. The detection molecule according to any of the preceding claims, wherein the oligonucleotide further comprises a handle for immobilization such as an additional oligonucleotide sequence, a free thiol function, biotin, avidin, azide, alkyne, a thioester, cysteine, an antibody or a hapten to an antibody.

10. The detection molecule according to any of the preceding claims, wherein the oligonucleotide further comprises a chemically or biologically active moiety that triggers a signal upon release of the oligonucleotide from the probe, such as a recognition site for a restriction enzyme, an mRNA portion, an antibody recognition site or an enzyme recognition site, which allow for a detectable binding and/or detectible reaction to occur.

11. The detection molecule according to any of the preceding claims, wherein the oligonucleotide has a length that is similar to the length of the stem forming portions of the probe, or shorter.

12. The detection molecule according to any of the preceding claims, wherein the oligonucleotide is longer than the stem forming portions of the probe, forming an overhang.

13. The detection molecule according to any of the preceding claims, wherein the fluorophore of the probe is FITC, TRITC, phycoerythrin, Cy7, FAM, Cy3, Cy3.5, Texas Red, LightCycler-Red 640, LightCycler Red 705, TMR, ROX, HEX, Cy5, Cy5.5, R6G, JA133, Alexa Fluor 488, Alexa Fluor 546, Alexa Fluor 633, Alexa Fluor 555, Alexa Fluor 647, fluorescent nanoparticles, or fluorescent transition metal complexes, such as europium.

14. The detection molecule according to any of the preceding claims, wherein the quencher of the probe is BHQ-1, BHQ-2, QSY-7, QSY-35, TRITC, Cy7, Cy3, Cy3.5, Texas Red, LightCycler-Red 640, LightCycler Red 705, TMR, ROX, HEX, Cy5, Cy5.5, the rhodamine derivative JA133, Alexa Fluor 546, Alexa Fluor 633, or Alexa Fluor 647, preferably Dabcyl.

15. The detection molecule according to any of the preceding claims, wherein the first and second stem forming portions of the probe comprise a poly(T) sequence and the oligonucleotide comprises a poly(a) sequence.

16. The detection molecule according to any of the preceding claims, wherein the first and the second stem forming portion of the probe and the oligonucleotide each comprise at least one base for oriented binding of the oligonucleotide to the probe, wherein the at least one base of the oligonucleotide interacts with the at least one base of the first and with the at least one base of the second stem forming portion of the probe.

17. A method for modulating the conformational constraint of a probe that comprises
- a first stem forming portion with a fluorophore for generating a detection signal,
- a second stem forming portion with a quencher for quenching the detection signal from the fluorophore when the quencher is in proximity to the fluorophore, whereby the first stem forming portion can form a stem structure with the second stem forming portion, and
- a loop forming portion located between the first and the second stem forming portion, for allowing the first stem forming portion to form a stem structure with the second stem forming portion,
**characterized by**
providing an oligonucleotide that comprises a portion that allows it to form a triplex structure with the first and second stem forming portions of the probe.

18. The method according to claim 17, whereby the length of the oligonucleotide is varied.

19. The method according to claim 17 or 18, whereby the melting temperature of the detection molecule formed by interaction of the oligonucleotide with the probe is determined to optimize sensitivity and/or selectivity of the detection molecule regarding the detection of a nucleic acid sequence, in particular of the detection of a single nucleotide polymorphism in a target nucleic acid.

20. Use of a detection molecule according to claims 1 to 16 for the detection of a nucleic acid sequence, in particular for detection of a single nucleotide polymorphism in a target nucleic acid.

21. Use according to claim 20, whereby the detection molecule is incubated with a target sequence and the detection signal generated upon binding of the probe caused by separation of the quencher and the fluorophore is detected.

22. A kit for detecting a nucleic acid sequence, in particular for detecting a single nucleotide polymorphism in a target nucleic acid, comprising
a) an oligonucleotide, and
b) a probe comprising
- a first stem forming portion with a fluorophore for generating a detection signal,
- a second stem forming portion with a quencher for quenching the detection signal from the fluorophore when the quencher is in proximity to the fluorophore, and
- a loop forming portion located between the first and the second stem forming portion, for allowing the first stem forming portion to form a stem structure together with the second stem forming portion of the probe and with the oligonucleotide,
whereby the oligonucleotide comprises a portion that allows it to form a triplex structure with the first and second stem forming portions of the probe.

23. A molecule according to SEQ ID NOs 1 to 24, in particular for detecting a nucleic acid sequence.
